# EUROPÄISCHE PATENTSCHRIFT

(11) **EP 3 421 381 B1**
(45) Veröffentlichungstag und Bekanntmachung des Hinweises auf die Patenterteilung: **28.08.2019**
(21) Anmeldenummer: 18163247.2
(22) Anmeldetag: 22.03.2018
(51) Int. Cl.: B65B 39/00, A61M 5/00

(54) **ZENTRIERVORRICHTUNG**
CENTERING DEVICE
DISPOSITIF DE CENTRAGE

(30) Priorität: 02.05.2017 DE 102017004233
(43) Veröffentlichungstag der Anmeldung: 02.01.2019
(73) Patentinhaber: Seidenader Maschinenbau GmbH, 85570 Markt Schwaben (DE)
(72) Erfinder: Grindinger, Herbert, 84453 Mühldorf/Inn (DE)
(74) Vertreter: Laufhütte, Dieter

(56) Entgegenhaltungen:
- EP-A2- 2 181 926
- US-A- 3 974 624

## Beschreibung

Die vorliegende Erfindung betrifft eine Zentriervorrichtung zum geordneten Einfügen von Objekten in einen Aufbewahrungsbehälter, auch Nest genannt. Insbesondere sind die in das Nest oder den Aufbewahrungsbehälter einzufügenden Objekte pharmazeutische Container, bspw. Spritzen, Ampullen, Zylinderampullen oder Vials.

Beim Einführen von Objekten in einen Aufbewahrungsbehälter kann es dazu kommen, dass der Vorgang aufgrund leicht deformierter Objekte (z.B. Nadelschutz) oder durch die relativ großen Toleranzen des Aufbewahrungsbehälters behindert wird. Dabei kommt erschwerend hinzu, dass die in dem Aufbewahrungsbehälter anzuordnenden Objekte überstehende Bestandteile (z.B. einen Fingergriff) aufweisen, wonach der zu zentrierende Durchmesser (z.B. Spritzenkörper) kleiner als der größte Durchmesser am Objekt (z.B. Fingergriff) ist.

Als weiter problematisch stellt sich die typische Anordnung der in dem Aufbewahrungsbehälter anzuordnenden Objekte dar, da diese in der Regel in versetzt zueinander angeordneten Reihen erfolgt, was eine exakte Seitwärtsbewegung einer Zentriervorrichtung erfordert, wenn mit Hilfe dieser die Reihe der anzuordnenden Objekte eingefügt wird.

Die vorliegend aufgeführten Nachteile aus dem Stand der Technik (s. z.B. US 3 974 624 A) werden mit Hilfe einer Zentriervorrichtung überwunden, die sämtliche Merkmale nach dem Anspruch 1 aufweist.

Demnach umfasst die erfindungsgemäße Zentriervorrichtung zum geordneten Einfügen von Objekten in einen Aufbewahrungsbehälter einen plattenartigen Grundkörper, mehrere zueinander benachbarte erste Einbuchtungen in einer Außenkante des Grundkörpers, eine Ausnehmung in den Grundkörper, und mehrere zueinander benachbarte zweite Einbuchtungen in einer Kante der Ausnehmung, wobei die ersten Einbuchtungen und die zweiten Einbuchtungen in ihrer Orientierung gleich sind.

Als Ausnehmung wird vorliegend eine von der Oberseite zur Unterseite des Grundkörpers verlaufende Öffnung angesehen, die einen im Grundkörper umlaufenden Randbereich aufweist. Die Öffnung ist also in ihrem Umfangsbereich vollständig von dem Grundkörper umgeben bzw. durch diesen definiert.

Mit Hilfe einer so ausgestalteten Zentriervorrichtung lassen sich die in den Aufbewahrungsbehälter einzufügenden Objekte besonders effektiv und schnell einbringen. Aufgrund der Buchtform der Einbuchtungen in ihren zugehörigen Kanten ist es möglich, Objekte mit einem zu zentrierenden Durchmesser einzufügen, der kleiner ist als der größte Durchmesser am Objekt. Dies kann dadurch geschehen, indem das zu zentrierende Objekt mit Hilfe der Einbuchtung zentriert wird und von dem zu zentrierenden Objekt seitlich weggeführt wird, so dass das im Nest angeordnete Objekt die Zentriervorrichtung aus der offenen Seite der Einbuchtung verlässt. Es kann vorgesehen sein, dass das zu zentrierende Objekt die Einbuchtung der Zentriervorrichtung nicht vollständig sondern nur zu einem Teil durchschreitet und, nachdem es angeordnet ist, aus einer zugehörigen Einbuchtung herausgeführt wird, indem eine Relativbewegung zwischen dem Objekt und der Zentriervorrichtung in eine Richtung vorgenommen wird, sodass das Objekt die Zentriervorrichtung über die offene Seite der Einbuchtung verlässt.

Nach einer optionalen Modifikation der Erfindung sind die ersten Einbuchtungen entlang einer ersten Geraden und die zweiten Einbuchtungen entlang einer zweiten Geraden angeordnet, wobei die erste Gerade und die zweite Gerade parallel zueinander sind.

Dadurch ergibt sich eine vorteilhafte Anordnung der mehreren Einbuchtungen, die ein besonders schnelles Befüllen eines Nests erlaubt.

Zudem kann vorgesehen sein, dass die ersten Einbuchtungen versetzt zu den zweiten Einbuchtungen angeordnet sind, vorzugsweise um eine halbe Breite der Einbuchtungen. Dadurch ist es möglich, dass hintereinander angeordnete Reihen, die versetzt zueinander angeordnet sind, mit der Zentriervorrichtung geschaffen werden. Der Versatz der ersten Einbuchtung gegenüber einer zweiten Einbuchtung beträgt dabei die Hälfte der Breite der Einbuchtungen. Ist die Außenkante zur der Kante der Ausnehmung parallel, sind die an diesen beiden Kanten angeordneten Einbuchtungen in der Kantenrichtung zueinander versetzt.

Vorzugsweise kann vorgesehen sein, dass die Anzahl der ersten Einbuchtungen der Anzahl der zweiten Einbuchtungen entspricht.

Nach einer weiteren Modifikation der Erfindung dient jede der ersten Einbuchtungen und jede der zweiten Einbuchtungen zum Einfügen eines Objektes in den Aufbewahrungsbehälter. Dabei sind die Abstände der ersten Einbuchtung untereinander bzw. der zweiten Einbuchtung untereinander gleich und auf den Aufbewahrungsbehälter abgestimmt, in dem die einzufügenden Objekte angeordnet werden.

Vorzugsweise führen mehrere benachbarte Einbuchtungen zu einer kammartigen Anordnung, bei der die benachbarten Einbuchtungen durch einen Steg voneinander getrennt sind.

Nach einer weiteren Modifikation der Erfindung verlaufen die Außenkante des Grundkörpers und die Kante der Ausnehmung in ihrer Dickenrichtung teilweise oder vollständig schräg. Mit anderen Worten sind die jeweiligen Kanten so angeschrägt, dass die offenen Seiten der Einbuchtungen bzw. der zwischen den Einbuchtungen angeordneten Stegen spitz von den Einbuchtungen weg auslaufen.

Nach einer weiteren Fortbildung der Erfindung bildet jede der Einbuchtungen in ihrer Draufsicht eine U-Form und/oder ist die Form der ersten Einbuchtungen gleich der Form der zweiten Einbuchtungen.

Nach einer weiteren optionalen Modifikation der Erfindung weist jede Einbuchtung einen von einer Oberseite zu einer Unterseite des Grundkörpers trichterförmigen Abschnitt auf, der von der Oberseite hin zur Unterseite hin verjüngend verläuft. Dadurch wird die Zentrierung eines abzulegenden Objekts hervorgerufen, da die Trichterform Abweichungen bezüglich des Sollortes des Ablegens ausgleicht.

Nach einer weiteren Fortbildung der Erfindung ist vorgesehen, dass ein von der Außenkante zur Kante der Ausnehmung verlaufender flächiger Bereich, der die ersten Einbuchtungen überdeckt, in seiner maximalen Dicke kleiner ist als ein die zweiten Einbuchtungen umfassender Bereich des Grundkörpers. Dies ist von Vorteil, wenn durch die Zentriervorrichtung gleichzeitig zwei Reihen von Objekten in den Aufbewahrungsbehälter eingeführt worden sind, wobei die beiden Reihen hintereinander angeordnet sind, und eine Relativbewegung der eingeführten Objekte in den Aufbewahrungsbehälter gegenüber der Zentriervorrichtung stattfindet. Dann ist möglich, dass aufgrund der geringeren Dicke, bzw. der stufenartigen Reduzierung der Dicke an der Unterseite der Zentriervorrichtung, bereits eingeführte Objekte in den Aufbewahrungsbehälter ohne Kontakt überfahren werden können, auch wenn nur eine geringe oder gar keine Abstandsvergrößerung in Höhenrichtung zwischen dem Nest und der Zentriervorrichtung ausgeführt worden ist.

Nach einer weiteren Fortbildung der Erfindung weist die Ausnehmung eine zweite Kante auf, die parallel zu der mit den zweiten Einbuchtungen versehenden Kante ist und in ihrer Dickenrichtung ebenfalls schräg verläuft, vorzugsweise mit gleicher Steigung und Orientierung wie die Außenkante und/oder die mit den zweiten Einbuchtungen versehene Kante der Ausnehmung.

Die Einbuchtungen weisen jeweils einen halbkreisförmigen Abschnitt auf, der in seinem Durchmesser von der Oberseite hin zur Unterseite geringer wird. Dabei ist eine offene Seite des halbkreisförmigen Abschnitts parallel zur der Kante angeordnet, an dem die Einbuchtung vorgesehen ist. Ferner weist eine jede Einbuchtung zwei Stege auf, die an jeder der Seiten des offenen Halbkreises geradlinig nach außen weg stehen. Die Stege sind im Wesentlichen senkrecht zur der Kante angeordnet, an dem die Einbuchtung vorgesehen ist.

Die Erfindung betrifft ferner einen Renester, der eine Zentriervorrichtung nach einer der vorstehend aufgeführten Varianten und einen Aufbewahrungsbehälter zum Aufbewahren von Objekten, insbesondere pharmazeutischen Behältern wie Spritzen, Ampullen, Zylinderampullen oder Vials umfasst.

Vorzugsweise ist die Zentriervorrichtung oberhalb des Aufbewahrungsbehälters angeordnet und ist dazu ausgelegt, beim Einfügen eines Objekts in den Aufbewahrungsbehälter das Objekt in Bezug auf einen Anordnungsplatz in dem Anordnungsbehälter zu zentrieren.

Hierzu kann vorteilhafterweise vorgesehen sein, dass die Teilung der Anordnungsplätze in dem Aufbewahrungsbehälter identisch ist zu der Teilung der mehreren Einbuchtungen einer Kante.

Nach einer weiteren Fortbildung des Renesters umfasst dieser eine Antriebseinheit, die dazu ausgelegt ist, eine Relativbewegung des Aufbewahrungsbehälters gegenüber der Zentriervorrichtung vorzusehen. Entweder durch Bewegen des Aufbewahrungsbehälters und/oder der Zentriervorrichtung.

Zudem kann vorgesehen sein, dass der Aufbewahrungsbehälter mehrere Reihen von zueinander versetzten Anordnungsplätzen für die einzufügenden Objekte aufweist, wobei vorzugsweise eine Antriebseinheit zum Ausführen einer Relativbewegung zwischen der Aufbewahrungseinheit und der Zentriervorrichtung vorgesehen ist, um unterschiedliche Reihen von Anordnungsplätzen so zu bewegen, dass diese mit den zugehörigen Einbuchtungen der Zentriervorrichtung fluchten.

Weitere Merkmale, Einzelheiten und Vorteile werden anhand der nachfolgenden Figurenbeschreibung ersichtlich. Dabei zeigen:
- Fig. 1:: eine Draufsicht auf die erfindungsgemäße Zentriervorrichtung,
- Fig. 2:: eine Draufsicht auf die Unterseite der erfindungsgemäßen Zentriervorrichtung,
- Fig. 3:: eine Perspektivansicht der erfindungsgemäßen Zentriervorrichtung,
- Fig. 4:: eine Schnittdarstellung der Zentriervorrichtung,
- Fig. 5:: eine Schnittdarstellung der Zentriervorrichtung bei der die nicht sichtbaren Elemente in gestrichelter Form wiedergegeben sind,
- Fig. 6:: eine perspektivische Darstellung einer geschnittenen Zentriervorrichtung, und
- Fig. 7:: einen Renester mit der erfindungsgemäßen Zentriervorrichtung.

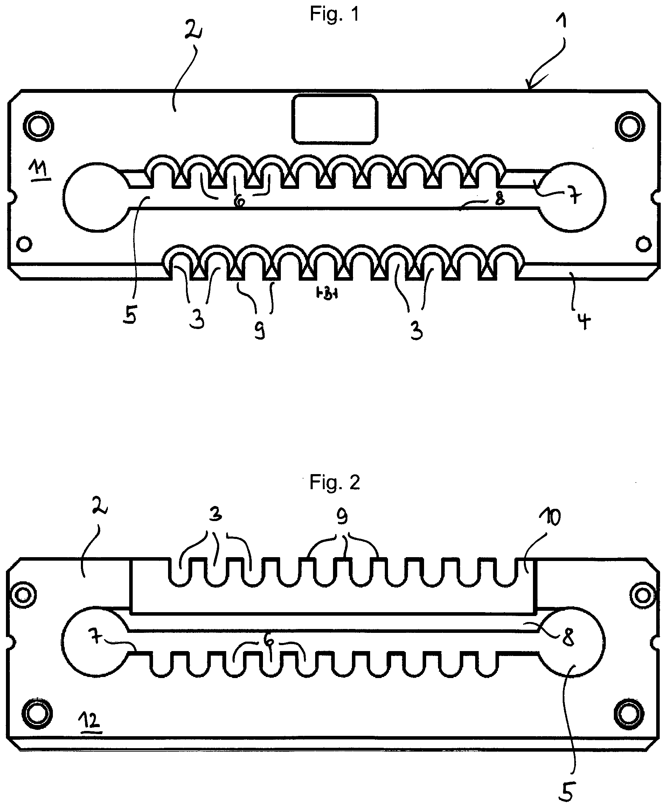

Fig. 1 zeigt eine Draufsicht auf die Oberseite 11 der Zentriervorrichtung 1. Der Grundkörper 2 weist dabei eine Außenkante 4 auf, in der mehrere Einbuchtungen 3 vorgesehen sind. Die Einbuchtungen 3 sind untereinander durch Stege 9 getrennt. Ferner erkennt man schematisch, dass die Außenkante 4 in Dickenrichtung schräg ist, sodass sie mit zunehmendem Abstand vom Grundkörper 2 in ihrer Dicke dünner wird.

Die Ausnehmung 5 in dem Grundkörper 2, der ebenfalls eine erste Kante 7 aufweist, ist parallel zur Außenkante 4. Hierin sind zweite Einbuchtungen 6 angeordnet, die in ihrer Form im Wesentlichen den ersten Einbuchtungen 3 entsprechen. Zudem ist die Orientierung der ersten Einbuchtungen 3 und der zweiten Einbuchtungen 6 identisch. Jedoch ist festzustellen, dass die zweiten Einbuchtungen 6 gegenüber den ersten Einbuchtungen 3 in der Längsrichtungen der Kanten 4, 7 versetzt angeordnet sind. Das Maß dieses Versatzes beträgt dabei die Hälfte der Breite B einer Einbuchtung, die ebenfalls in Längsrichtung der Kante gemessen wird.

Zudem erkennt man beim Blick auf die Oberseite 11 der Zentriervorrichtung 1 die Trichterform der Einbuchtungen 3, 6. Dadurch ist es möglich, ein Objekt mit etwas geringerer Genauigkeit in die Trichterform hereinzuführen und durch die gezielte Ablenkung der Trichterform der Einbuchtungen 3, 6 mit einer hohen Positionsgenauigkeit an einen vorbestimmten Ort anzuordnen.

Wurden beispielsweise mehrere von einem Greifelement getragene Spritzen aufgenommen, so kommt es oftmals vor, dass ein Nadelschutz nicht vollkommen orthogonal zu der darin aufgenommenen Nadel angeordnet ist und das Ablegen in einem Nest bzw. in einem dafür vorgesehen Anordnungsplatz des Nests nicht zentriert vorgenommen werden kann. Demnach muss ohne die Vorrichtung 1 eine Arbeitsgeschwindigkeit des Greifelements beim Übergeben der mehreren Spritzen an einen Aufbewahrungsbehälter (z.B. ein Nest) abgesenkt werden, um die Spritzen zuverlässig an das Nest mit sehr hoher Genauigkeit zu übergeben.

Ferner weist eine solche Spritze bzw. ein solches Objekt überstehende Bestandteile auf, sodass ein vollständiges Hindurchtreten des anzuordnenden Objekts durch die Einbuchtung nicht möglich ist. Jedoch ist es möglich, die Zentriervorrichtung gegenüber dem angeordneten Objekt zu bewegen, so dass das Objekt aus der offenen Seite der Einbuchtung bewegt wird. Diese kammartige Ausführung der mehreren aneinandergereihten Einbuchtungen vereinfacht ein Ablegeverfahren beträchtlich.

Um zu verhindern, dass sich Objekte, die nach dem Herausbewegen aus der Zentrierhilfe nicht komplett in das Nest hineingleiten, verklemmen, sind die Einbuchtungen der Zentrierhilfe abgeschrägt und zwingen somit bei einer Bewegung die Objekte in ihre korrekte Position.

Zudem verhindert die versetze Anordnung der beiden Reihen von Einbuchtungen die Notwendigkeit eines lateralen Verschiebens der Zentriervorrichtung beim Bestücken eines Nests aufgrund der versetzt angeordneten Anordnungsplätze. Dadurch wird eine weitere Fehlerquelle eliminiert, die zu einer deutlichen Steigerung der Bearbeitungsgeschwindigkeit führt.

Fig. 2 zeigt die Unterseite 12 der Zentriervorrichtung 1 und man erkennt, dass auch die zweite Kante 8 der Ausnehmung 5 entsprechend der Außenkante 4 und der ersten Kante 7 der Ausnehmung in Dickenrichtung des Grundkörpers 2 schräg verläuft. Bei der Ansicht von unten erkennt man, dass die Einbuchtungen eine Trichterform aufweisen, sodass darin von oben eingeführte Elemente zielsicher in einen bestimmten Bereich positioniert werden können. Weiter erkennt man auch die zwischen den einzelnen Einbuchtungen angeordneten Stege, die zusammen mit den Einbuchtungen 3, 6 eine U-Form bilden.

Die flächige Unterseite 12 des Grundkörpers 2 stufenartig besitzt einen abgesetzten Bereich 10, der die ersten Einbuchtungen 3 von der Außenkante 4 bis hin zur zweiten Kante 8 der Ausnehmung 5 umfasst. Dieser Bereich ist in gegenüber der übrigen Unterseite 12 stufenartig abgesetzt (vgl. Stufen 17), so dass vorzugsweise die Fläche des Bereichs 10 eine geringere Dicke aufweist. Dadurch wir das Überfahren von bereits in einem Aufnahmebehälter angeordneten Objekten erleichtert, die ansonsten eventuell an die Oberseite 11 anstoßen.

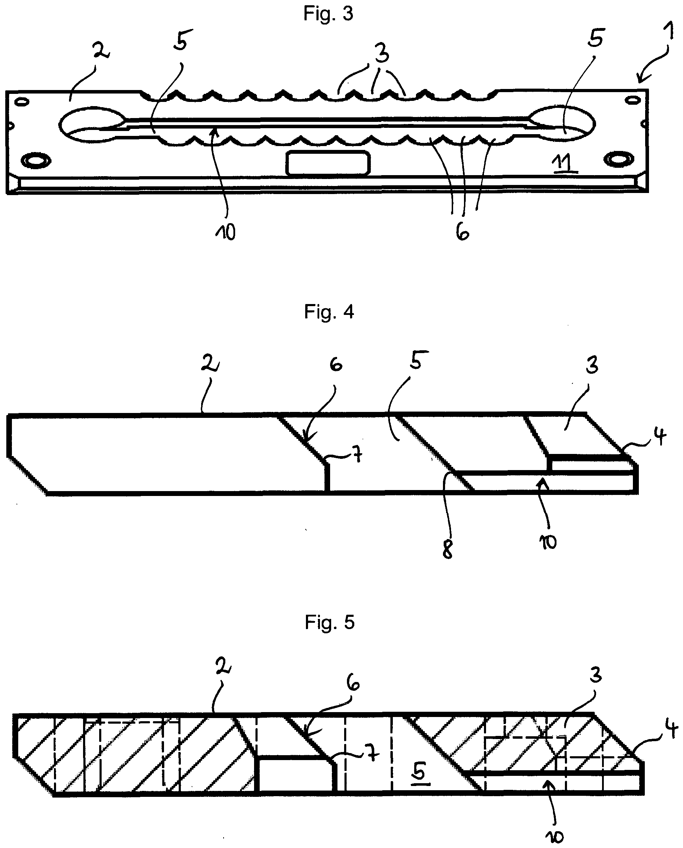

Fig. 3 ist eine perspektivische Darstellung der erfindungsgemäßen Zentriervorrichtung 1 aus einer Rückansicht. Auch hier ist erneut der Bereich 10 zu sehen, der durch die geringere Dicke gegenüber den anderen Bereichen der Zentriervorrichtung 1 gekennzeichnet ist.

Fig. 4 zeigt eine Schnittdarstellung der erfindungsgemäßen Zentriervorrichtung 1. Man erkennt den Grundkörper 2, sowie die angeschrägten Kanten 7, 8 und 4.

Fig. 5 zeigt eine weitere Darstellung einer Schnittansicht der erfindungsgemäßen Zentriervorrichtung, bei der verdeckte Elemente in gestrichelter Form dargestellt sind.

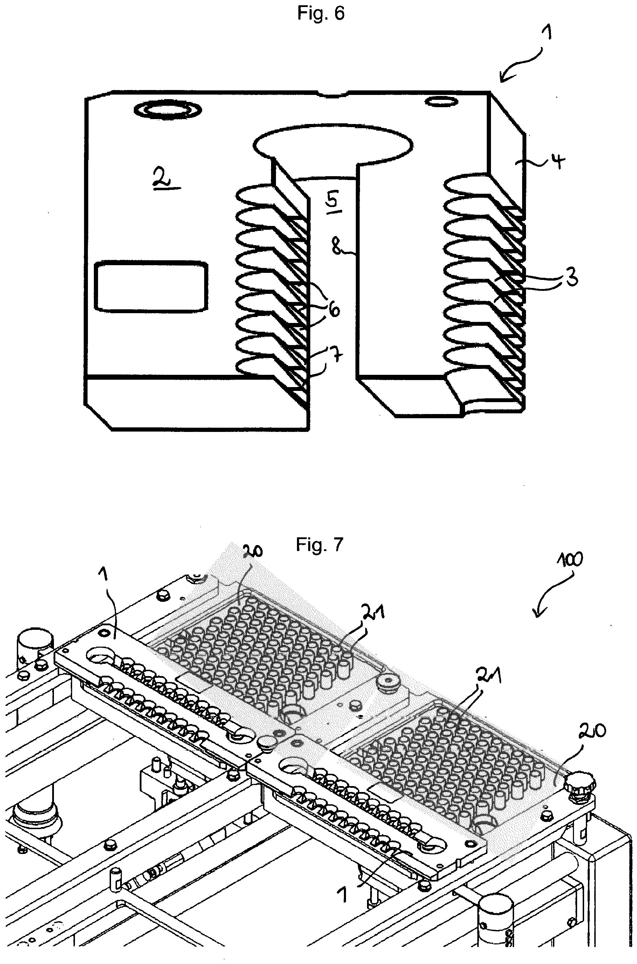

Fig. 6 zeigt eine perspektivische Ansicht der in Fig. 5 dargestellten Schnittansicht, anhand derer der Aufbau des Grundkörpers 2 deutlich wird. Hier kann man auch gut den Bereich 10 erkennen, der gegenüber der flächigen Unterseite 12 über eine Stufe 17 in Richtung Oberseite 11 abgesetzt ist.

Fig. 7 zeigt einen Renester, der mit der Zentriervorrichtung 1 nach der Erfindung versehen ist. Man erkennt, dass die Zentriervorrichtung 1 oberhalb eines Aufbewahrungsbehältnisses 20 angeordnet ist, sodass das Einfügen eines Objektes in den dafür vorgesehen Anordnungsplatz 21 mit Hilfe einer zugehörigen Einbuchtung 3, 6 der Zentriervorrichtung 1 vorgenommen wird. Ist eine Reihe vollständig mit den Objekten gefüllt, bewegt sich entweder die Zentriervorrichtung 1 oder das Aufbewahrungsbehältnis 20, so dass im Anschluss an diese Bewegung die Einbuchtungen mit noch leeren Anordnungsplätzen 21 fluchten.

Dadurch ist es möglich, das Anordnungsverfahren zum Einbringen der Objekte in die dafür vorgesehenen Anordnungsplätze 21 schneller ablaufen zu lassen. Dieser Gewinn der Maschinengeschwindigkeit bewirkt eine insgesamt bessere Maschineneffizienz und führt zu einem geringeren Energieverbrauch bei einem gleichen Durchsatz.

## Patentansprüche

1. Zentriervorrichtung (1) zum geordneten Einfügen von Objekten in einen Aufbewahrungsbehälter (20), umfassend:
einen plattenartigen Grundkörper (2),
mehrere zueinander benachbarte erste Einbuchtungen (3) in einer Außenkante (4) des Grundkörpers (2), **gekennzeichnet durch** eine Ausnehmung (5) in dem Grundkörper (2), und
mehrere zueinander benachbarte zweite Einbuchtungen (6) in einer Kante (7) der Ausnehmung (5),
wobei die ersten Einbuchtungen (3) und die zweiten Einbuchtungen (6) in ihrer Orientierung gleich sind.

2. Vorrichtung (1) nach Anspruch 1, wobei
die ersten Einbuchtungen (3) entlang einer ersten Geraden angeordnet sind,
die zweiten Einbuchtungen (6) entlang einer zweiten Geraden angeordnet sind, und
die erste Gerade und die zweite Gerade parallel zueinander sind.

3. Vorrichtung (1) nach einem der vorhergehenden Ansprüche, wobei die ersten Einbuchtungen (3) zu den zweiten Einbuchtungen (6) versetzt angeordnet sind, vorzugsweise um eine halbe Breite (B) der Einbuchtungen (3, 6).

4. Vorrichtung (1) nach einem der vorhergehenden Ansprüche, wobei die Anzahl der ersten Einbuchtungen (3) der Anzahl der zweiten Einbuchtungen (6) entspricht.

5. Vorrichtung (1) nach einem der vorhergehenden Ansprüche, wobei jede der ersten Einbuchtungen (3) und jeder der zweiten Einbuchtungen (6) zum Einfügen eines Objekts in den Aufbewahrungsbehälter dient.

6. Vorrichtung (1) nach einem der vorhergehenden Ansprüche, wobei die Außenkante (4) des Grundkörpers (2) und die Kante (7) der Ausnehmung (5) teilweise oder vollständig schräg verlaufen, vorzugsweise von einer Oberseite (11) des Grundkörpers (2) zu einer von den Einbuchtungen (3, 6) abgewandten Seite hin zu einer Unterseite (12) des Grundkörpers (2).

7. Vorrichtung (1) nach einem der vorhergehenden Ansprüche, wobei mehrere benachbarte Einbuchtungen (3, 6) zu einer kammartigen Anordnung führen, bei der die benachbarten Einbuchtungen (3, 6) durch einen Steg (9) voneinander getrennt sind.

8. Vorrichtung (1) nach einem der vorhergehenden Ansprüche, wobei jede der Einbuchtungen (3, 6) in ihrer Draufsicht eine U-Form bildet und/oder die Form der ersten Einbuchtungen (3) gleich der Form der zweiten Einbuchtungen (6) ist.

9. Vorrichtung (1) nach einem der vorhergehenden Ansprüche, wobei von einer Oberseite (11) zu einer Unterseite (12) des Grundkörpers (2) jede Einbuchtung (3, 6) einen trichterförmigen Abschnitt aufweist, der von der Oberseite (11) hin zur Unterseite (12) verjüngend verläuft.

10. Vorrichtung (1) nach einem der vorhergehenden Ansprüche, wobei ein von der Außenkante (4) zur Kante (7) der Ausnehmung (5) verlaufender Bereich (10), der zudem die ersten Einbuchtungen (3) umfasst, in seiner maximalen Dicke (D) kleiner ist als ein die zweiten Einbuchtungen (6) umfassender Bereich des Grundkörpers (2).

11. Vorrichtung (1) nach einem der vorhergehenden Ansprüche, wobei die Ausnehmung (5) eine zweite Kante (8) aufweist, die parallel zu der mit den zweiten Einbuchtungen (6) versehenen Kante (7) ist und schräg verläuft, vorzugsweise mit gleicher Steigung und Orientierung wie die Außenkante (4) und/oder die mit den zweiten Einbuchtungen (6) versehene Kante (7) der Ausnehmung (5).

12. Renester (100), umfassend:
eine Zentriervorrichtung (1) nach einem der vorhergehenden Ansprüche, und einen Aufbewahrungsbehälter (20) zum Aufbewahren von Objekten, insbesondere pharmazeutischen Behältern wie Spritzen, Ampullen, Zylinderampullen oder Vials.

13. Renester (100) nach Anspruch 12, wobei die Zentriervorrichtung (1) oberhalb des Aufbewahrungsbehälters (20) angeordnet ist und dazu dient, beim Einfügen eines Objekts in den Aufbewahrungsbehälter (20) das Objekt in Bezug auf seinen Anordnungsplatz (21) in dem Aufbewahrungsbehälter (20) zu zentrieren.

14. Renester (100) nach einem der Ansprüche 12 oder 13, ferner umfassend eine Antriebseinheit, die dazu ausgelegt ist, den Aufbewahrungsbehälter (20) gegenüber der Zentriervorrichtung (1) zu bewegen.

15. Renester (100) nach einem der vorhergehenden Ansprüche 12 bis 14, wobei der Aufbewahrungsbehälter (20) mehrere Reihen von zueinander versetzten Anordnungsplätzen (21) für die Objekte aufweist, und wobei vorzugsweise eine Antriebseinheit zum Ausführen einer Relativbewegung zwischen der Aufbewahrungseinheit (20) und der Zentriervorrichtung (1) vorgesehen ist, um unterschiedliche Reihen von Anordnungsplätzen (21) so zu bewegen, dass diese mit den zugehörigen Einbuchtungen (3, 6) der Zentriervorrichtung (1) fluchten.

## Claims

1. A centering apparatus (1) for the orderly insertion of objects into a storage container (20) comprising:
a plate-like base body (2);
a plurality of mutually adjacent first indentations (3) in an outer edge (4) of the base body (2);
**characterized by**
a cut-out (5) in the base body (2); and
a plurality of mutually adjacent second indentations (6) in an edge (7) of the cut-out (5),
with the orientations of the first indentations (3) and the second indentations (6) being the same.

2. An apparatus (1) in accordance with claim 1, wherein
the first indentations (3) are arranged along a first straight line;
the second indentations (6) are arranged along a second straight line; and
the first straight line and the second straight line are in parallel with one another.

3. An apparatus (1) in accordance with one of the preceding claims, wherein the first indentations (3) are arranged offset from the second indentations (6), preferably by half a width (B) of the indentations (3, 6).

4. An apparatus (1) in accordance with one of the preceding claims, wherein the number of first indentations (3) corresponds to the number of second indentations (6).

5. An apparatus (1) in accordance with one of the preceding claims, wherein each of the first indentations (3) and each of the second indentations (6) serves the insertion of an object into the storage container.

6. An apparatus (1) in accordance with one of the preceding claims, wherein the outer edge (4) of the base body (2) and the edge (7) of the cut-out (5) extend partly or completely in a slanted manner, preferably from an upper side (11) of the base body (2) to a side remote from the indentations (3, 6) toward a lower side (12) of the base body (2).

7. An apparatus (1) in accordance with one of the preceding claims, wherein a plurality of adjacent indentations (3, 6) produce a comb-like arrangement in which the adjacent indentations (3, 6) are separated from one another by a web (9).

8. An apparatus (1) in accordance with one of the preceding claims, wherein each of the indentations (3, 6) forms a U shape in its plan view; and/or wherein the shape of the first indentations (3) is the same as the shape of the second indentations (6).

9. An apparatus (1) in accordance with one of the preceding claims, wherein each indentation (3, 6) has a funnel-like section from an upper side (11) to a lower side (12) of the base body (2) that extends in a tapering form from the upper side (11) to the lower side (12).

10. An apparatus (1) in accordance with one of the preceding claims, wherein the maximum thickness (D) of a region (10) that extends from the outer edge (4) to the edge (7) of the cut-out (5) and that additionally comprises the first indentations (3) is smaller than a region of the base body (2) comprising the second indentations (6).

11. An apparatus (1) in accordance with one of the preceding claims, wherein the cut-out (5) has a second edge (8) that is in parallel with the edge (7) provided with the second indentations (6) and that extends in a slanted manner, preferably with the same gradient and orientation as the outer edge (4) and/or the edge (7) of the cut-out (5) provided with the second indentations (6).

12. A renester (100) comprising:
a centering apparatus (1) in accordance with one of the preceding claims; and
a storage container (20) for storing objects, in particular pharmaceutical containers such as syringes, ampoules, syringe cartridges, or vials.

13. A renester (100) in accordance with claim 12, wherein the centering apparatus (1) is arranged above the storage container (20) and serves to center the object with respect to its arrangement position (21) in the storage container (20) on the insertion of an object into the storage container (20).

14. A renester (100) in accordance with one of the claims 12 or 13 further comprising a drive unit that is configured to move the storage container (20) with respect to the centering apparatus (1).

15. A renester (100) in accordance with one of the preceding claims 12 to 14, wherein the storage container (20) has a plurality of rows of mutually offset arrangement positions (21) for the objects; and wherein a drive unit is preferably provided for carrying out a relative movement between the storage unit (20) and the centering apparatus (1) to move different rows of arrangement positions (21) such that they align with the associated indentations (3, 6) of the centering apparatus (1).

## Revendications

1. Dispositif de centrage (1) destiné à l'introduction ordonnée d'objets dans un contenant de stockage (20), comprenant :
un corps de base (2) en forme de plaque,
plusieurs premiers creux (3) adjacents les uns aux autres dans un bord extérieur (4) du corps de base (2),
**caractérisé par**
un évidement (5) dans le corps de base (2), et
plusieurs seconds creux (6) adjacents les uns aux autres dans un bord (7) de l'évidement (5),
les premiers creux (3) et les seconds creux (6) étant orientés de manière identique.

2. Dispositif (1) selon la revendication 1, dans lequel les premiers creux (3) sont disposés le long d'une première ligne droite, les seconds creux (6) sont disposés le long d'une seconde ligne droite, et la première ligne droite et la seconde ligne droite sont parallèles l'une à l'autre.

3. Dispositif (1) selon l'une des revendications précédentes, dans lequel les premiers creux (3) sont disposés décalés par rapport aux seconds creux (6), de préférence d'une demi-largeur (B) des creux (3, 6).

4. Dispositif (1) selon l'une des revendications précédentes, dans lequel le nombre des premiers creux (3) correspond au nombre des seconds creux (6).

5. Dispositif (1) selon l'une des revendications précédentes, dans lequel chacun des premiers creux (3) et chacun des seconds creux (6) sert à introduire un objet dans le contenant de stockage.

6. Dispositif (1) selon l'une des revendications précédentes, dans lequel le bord extérieur (4) du corps de base (2) et le bord (7) de l'évidement (5) s'étendent en partie ou entièrement de manière oblique, de préférence d'une face supérieure (11) du corps de base (2) à une face inférieure (12) du corps de base (2) en direction d'un côté orienté dans le sens opposé aux creux (3, 6).

7. Dispositif (1) selon l'une des revendications précédentes, dans lequel plusieurs creux (3, 6) adjacents conduisent à une disposition en forme de peigne, dans laquelle les creux (3, 6) adjacents sont séparés les uns des autres par une dent (9).

8. Dispositif (1) selon l'une des revendications précédentes, dans lequel chacun des creux (3, 6) présente une forme de U en vue de dessus et/ou la forme des premiers creux (3) est identique à la forme des seconds creux (6).

9. Dispositif (1) selon l'une des revendications précédentes, dans lequel, d'une face supérieure (11) à une face inférieure (12) du corps de base (2), chaque creux (3, 6) comporte une partie en forme d'entonnoir, qui s'amincit de la face supérieure (11) à la face inférieure (12).

10. Dispositif (1) selon l'une des revendications précédentes, dans lequel une zone (10) s'étendant du bord extérieur (4) au bord (7) de l'évidement (5), qui comprend en outre les premiers creux (3), présente une épaisseur maximale (D) inférieure à une zone du corps de base (2) comprenant les seconds creux (6).

11. Dispositif (1) selon l'une des revendications précédentes, dans lequel l'évidement (5) comporte un second bord (8), qui est parallèle au bord (7) pourvu des seconds creux (6) et qui s'étend de manière oblique, de préférence avec une inclinaison et une orientation identiques au bord extérieur (4) et/ou au bord (7) de l'évidement (5) pourvu des seconds creux (6).

12. Renester (100), comprenant :
un dispositif de centrage (1) selon l'une des revendications précédentes, et un contenant de stockage (20) destiné au stockage d'objets, en particulier de contenants pharmaceutiques tels que des seringues, des ampoules, des carpules ou des fioles.

13. Renester (100) selon la revendication 12, dans lequel le dispositif de centrage (1) est disposé au-dessus du contenant de stockage (20) et, lors de l'introduction d'un objet dans le contenant de stockage (20), sert à centrer l'objet par rapport à son emplacement (21) dans le contenant de stockage (20).

14. Renester (100) selon l'une des revendications 12 ou 13, comprenant en outre une unité d'entraînement, qui est conçue pour déplacer le contenant de stockage (20) par rapport au dispositif de centrage (1).

15. Renester (100) selon l'une des revendications 12 à 14, dans lequel le contenant de stockage (20) comporte plusieurs rangées d'emplacements (21) décalés les uns par rapport aux autres pour les objets, et dans lequel une unité d'entraînement est de préférence prévue pour effectuer un déplacement relatif entre l'unité de stockage (20) et le dispositif de centrage (1), afin de déplacer différentes rangées d'emplacements (21) de telle sorte que ceux-ci s'alignent avec les creux (3, 6) associés du dispositif de centrage (1).
